# FASCICULE DE BREVET EUROPEEN

(11) **EP 1 845 781 B1**
(45) Date de publication et mention de la délivrance du brevet: **09.07.2008**
(21) Numéro de dépôt: 06755417.0
(22) Date de dépôt: 10.02.2006
(51) Int. Cl.: A01N 43/54, A61K 31/513

(54) **UTILISATION DE COMPOSES DERIVES DE PYRIMIDINETRIONE EN TANT QU'INHIBITEURS DE L'ACETYLCHOLINESTERASE, COMPOSITIONS CONTENANT CES DERIVES ET LEURS UTILISATIONS**
VERWENDUNG VON PYRIMIDINETRION-DERIVATEN ALS ACETYLCHOLINESTERASE-HEMMER, DIESE ENTHALTENDE ZUSAMMENSETZUNGEN UND DEREN VERWENDUNGEN
USE OF COMPOUNDS DERIVED FROM PYRIMIDINETRIONE AS ACETYL CHOLINESTERASE INHIBITORS, COMPOSITIONS CONTAINING SAID DERIVATIVES, AND THE USES THEREOF

(30) Priorité: 10.02.2005 FR 0501328
(43) Date de publication de la demande: 24.10.2007
(73) Titulaire: Centre National de la Recherche Scientifique, 75016 Paris (FR); Université de Montpellier II, 34095 Montpellier Cedex 5 (FR)
(72) Inventeur: WEILL, Mylène, F-34090 Montpellier (FR); FORT, Philippe, F-34170 Castelnau Le Lez (FR); LEONETTI, Jean-Paul, F-34170 Castelnau Le Lez (FR)
(74) Mandataire: Corizzi, Valérie
(86) Numéro de dépôt international: PCT/FR2006/000307
(87) Numéro de publication internationale: WO 2006/097588

(56) Documents cités:
- EP-A- 0 382 112
- EP-A- 0 455 300

## Description

La présente Invention est relative à l'utilisation de composés dérivés de pyrimidinetrione de formule générale (I) telle que définie ci-après en tant qu'inhibiteurs de l'acétylcholinestérase (AChE), à leur utilisation en tant qu'insecticides, ainsi qu'aux compositions contenant ces dérivés.

De nombreuses régions du monde pratiquent un contrôle intensif des insectes par traitement massif à l'aide d'insecticides, ce qui a progressivement conduit à l'émergence de populations d'insectes résistants aux traitements.

L'émergence de populations d'insectes résistants aux insecticides couramment utilisés représente un problème de santé publique important dans la mesure où certains insectes sont les vecteurs de la transmission de maladies à l'homme comme par exemple les moustiques transmettant le paludisme (*Anopheles gambiae*), la fièvre jaune et la dengue *(Aedes aegypti*) ou des encéphalopathies virales *(Culex tritaeniorhynchus* et *Culex pipiens*).

Parmi les insecticides les plus utilisés, on peut citer les organophosphorés et les carbamates. La cible de ces deux principales familles d'insecticides est l'acétylcholinestérase (AChE), enzyme essentielle qui hydrolyse l'acétylcholine dans les synapses cholinergiques. L'inhibition de l'AChE empêche la désactivation du signal synaptique, conduisant ainsi à une perte de contrôle de la transmission cholinergique.

Parmi les mécanismes de résistance, la sélection de mutations rendant l'AChE insensible aux insecticides organophosphorés et carbamates a été observée dans de nombreux cas (Anazawa Y., Insect Biochem Mol Biol, 2003, 33(5), 509-514; Fallang A., Pest Manag Sci, 2004, 60(12), 1163-1170 ; Li F., Insect Biochem Mol Biol, 2004, 34(4), 397-405 ; Menozzi P., BMC Evol Biol, 2004, 4(1), 4 ; Nabeshima T., Biochem Biophys Res Commun, 2004, 313(3), 794-801 ; Vontas JG., Insect Mol Biol, 2002, 11(4), 329-336; Walsh SB., Biochem J, 2001, 359(Pt1), 175-181).

Les inventeurs ont ainsi précédemment identifié chez les Culicidés (Moustiques) le gène codant l'AChE, cible des organophosphorés et des carbamates. De plus, la même et unique mutation (Glycine vers Serine en position 119 selon la nomenclature AChE *Torpedo*) a été retrouvée dans toutes les populations de plusieurs espèces de moustiques qui présentent un haut niveau de résistance aux organophosphorés et aux carbamates, et en particulier chez *Culex pipiens, Anopheles gambiae* et *Anopheles albimanus*, à savoir les principaux vecteurs de la fièvre jaune, de la malaria et d'encéphalopathies virales (Weill et al., Insect Molecular Biology, 2004, 13(1), 1-7 ; Weill et al., Nature, 2003, 423, 136-137).

Compte tenu de ce qui précède, il apparaît donc que la mise au point de nouvelles stratégies de lutte contre les insectes représente un enjeu sanitaire et économique crucial.

Pour contourner ces résistances établies, nombreux sont ceux qui cherchent à diversifier les cibles biologiques. Cependant, si une telle diversification est souhaitable sur le long terme, elle n'empêchera pas l'apparition de populations résistantes et nécessitera un effort important pour atteindre le niveau de connaissances déjà acquises sur les cibles actuellement visées.

Une autre possibilité consiste à chercher des molécules efficaces sur les formes résistantes des cibles déjà validées. L'avantage majeur de cette approche est la plus faible probabilité que les cibles acceptent une mutation supplémentaire sans entraîner un effet délétère dû à une réduction de leur activité.

Les Inventeurs ont fait le choix de cette deuxième approche et se sont donné pour but de trouver de nouveaux insecticides capables de tuer les populations d'insectes ayant développé une résistance aux insecticides conventionnels.

Ainsi, les Inventeurs ont identifié une famille de composés capable d'inhiber l'AChE, et de façon préférentielle d'agir sur l'AChE insensible aux organophosphorés et aux carbamates. De tels composés possèdent donc des propriétés insecticides et sont capable d'agir notamment de manière préférentielle sur des populations d'insectes qui ont développé une résistance aux insecticides conventionnels.

Ainsi, les composés selon la présente Invention peuvent être avantageusement utilisés pour contrôler le développement de populations résistantes aux organophosphorés et aux carbamates. De plus, compte tenu de la réduction de 40% de l'activité spécifique occasionnée par la première mutation, il est peu probable que l'AChE conserve une activité suffisante à la suite d'une mutation supplémentaire. L'utilisation des composés insecticides de ce type permettrait donc d'empêcher l'extension des espèces actuellement résistantes tout en minimisant les risques d'apparition de nouvelle résistance à ces nouveaux composés.

Par ailleurs, la structure de ces composés permet par exemple par la chimie combinatoire, d'obtenir un grand nombre de variants de ces composés, ce qui devrait permettre de les adapter rapidement à l'émergence d'éventuelles résistances ultérieures. Ces molécules sont également relativement instables en milieu aqueux ce qui est un avantage pour la protection de l'environnement (faible rémanence).

Selon un premier aspect, l'Invention a pour objet l'utilisation d'au moins un composé dérivé de pyrimidinetrione en tant qu'inhibiteur de l'AchE, ledit composé répondant à la formule générale (I) suivante : 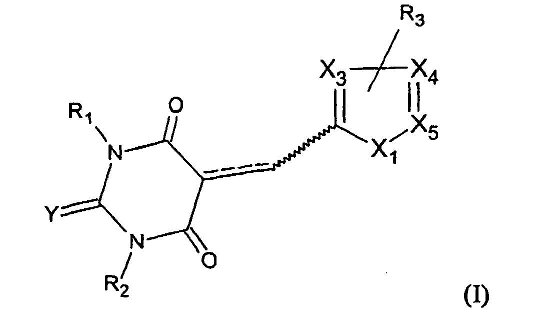 dans laquelle :
---- représente une liaison qui peut être une liaison simple ou une liaison double;
R₁ représente un groupe choisi parmi un atome d'hydrogène et un groupe alkyle, saturé ou insaturé, linéaire, ramifié ou cyclique, en C₁-C₆, éventuellement substitué par un groupe hydroxyle ou un atome d'halogène ;
R₂ représente un groupe choisi parmi un groupe aryle éventuellement substitué et un groupe alkyle, saturé ou insaturé, linéaire, ramifié ou cyclique, en C₁-C₄, éventuellement substitué par un ou plusieurs groupements choisis parmi les atomes d'halogène, les groupements hydroxyles, les hétérocycles comprenant 1 à 5 atomes de carbone et 1 à 5 hétéro-atomes tels que l'oxygène, le soufre et l'azote ;
R₃ peut être en position 3, 4 ou 5 du cycle et représente un groupe choisi parmi un atome d'hydrogène et un groupe alkyle, saturé ou insaturé, linéaire, ramifié ou cyclique, en C₁-C₄, éventuellement substitué par un groupe hydroxyle ou un atome d'halogène ;
X₁ représente un atome choisi parmi le soufre, l'azote et l'oxygène ;
X₃, X₄ et X₅, identiques ou différents, sont choisis parmi un atome de carbone et un atome d'azote, deux atomes au plus parmi X₃, X₄ et X₅ étant des atomes d'azote ; et
Y représente un atome choisi parmi l'oxygène et le soufre.

Lorsque R₂ est un groupe aryle substitué, il est substitué par un groupe alkyle ou alkoxy en C₁-C₄, comme par exemple un groupement méthyle, éthyle, n-propyle, isopropyle, n-butyle, terbutyle, isobutyle, un groupement méthoxy, éthoxy, isopropoxy, butoxy. Il peut également être substitué par un groupement hydroxyle ou un atome d'halogène choisi parmi le chlore, le brome, l'iode et le fluor, et en particulier le chlore. Par ailleurs, la substitution du groupe aryle peut avoir lieu en position ortho, méta ou para, et de préférence en méta ou para.

Parmi les cycles 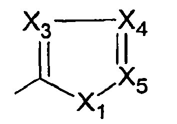 qui sont utilisables dans la formule (I) ci-dessus, on peut citer notamment : 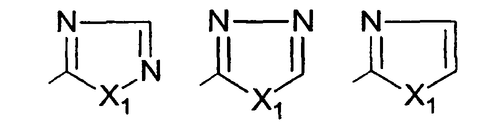 et 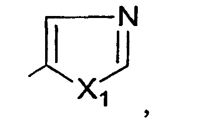 X₁ ayant la même définition que ci-dessus.

La Demande de Brevet DE 3903404 divulgue des dérivés de pyrimidinetrione utilisés comme insecticides et anthelmintiques notamment.

Le Brevet Européen EP 0455300 divulgue des dérivés de l'acide barbiturique et leur utilisation comme insecticides ; les cibles visées étant distinctes des moustiques.

Toutefois, les composés décrits dans ces deux documents sont distincts de ceux répondant à la formule générale (I) telle que définie ci-dessus. En outre, aucun des documents de l'art antérieur ne suggère ni ne mentionne une activité d'inhibiteur de l'AchE, en particulier d'inhibiteur d'une forme d'AchE résistante aux insecticides organophosphorés et carbamates.

Selon une forme préférée de l'Invention, lorsque R₁ représente un groupe alkyle en C₁-C₆, celui-ci est de préférence un groupe alkyle en C₁-C₄, et de manière encore préférée un groupe méthyle. Ainsi, parmi les composés préférés selon l'Invention, on peut en particulier citer les composés dans lesquels R₁ est choisi parmi un atome d'hydrogène et un groupe méthyle.

Selon une autre forme préférée de l'Invention, R₂ représente un groupe aryle, éventuellement substitué par un groupement choisi parmi un alkyle en C₁-C₄ et un atome d'halogène, et avantageusement un phényle éventuellement substitué par un groupement choisi parmi un alkyle en C₁-C₄ et un atome d'halogène.

Parmi les composés préférés selon l'Invention, on peut en particulier citer les composés dans lesquels R₂ est choisi parmi un méthyle, un allyle, un groupement furyl éventuellement substitué par un méthyle, un butyle, un phényle non substitué, un phényle substitué par un groupe méthyle en position méta, un phényle substitué par un groupe méthyle en position para, un phényle di-substitué en position méta et para par un méthyle, un phényle substitué par un éthyle en position para, un phényle substitué par un groupe méthoxy en position méta, un phényle substitué par un groupe méthoxy en position para, un phényle substitué par un éthoxy en position para, un phényle substitué par un isopropyle en position para, un phényle substitué par un atome de chlore en position para, un phényle substitué par un atome de brome en position para, un phényle substitué par un atome de fluor en position para. Dans les composés préférés selon l'Invention, Y représente avantageusement un atome d'oxygène.

Selon une forme préférée de l'Invention, X₁ est choisi parmi l'oxygène et le soufre.

De façon avantageuse, les composés selon l'Invention sont des composés dans lesquels X₃, X₄ et X₅ sont identiques et représentent tous un atome de carbone.

Parmi les composés préférés selon l'Invention, on peut citer en particulier les composés dans lesquels R₃ est choisi parmi un atome d'hydrogène et un groupe méthyle. De plus, R₃ est avantageusement en position 5 du cycle furanne, thiophène ou pyrrole.

Parmi les composés de formule (I) ci-dessus, on peut en particulier citer les composés préférés suivants énumérés dans le tableau I :

**TABLEAU I**

| Composés | Noms |
|---|---|
| (1) | 5-[(5-méthyl-2-furyl)méthylène]-1-phényl-2,4,6(1H,3H,5H)-pyrimidinetrione |
| (2) | 1-(3-méthylphényl)-5-[(5-méthyl-2-thienyl)méthylène]-2,4,6(1H,3H,5H)-pyrimidinetrione |
| (3) | 1-(3-méthoxyphenyl)-5-[(5-méthyl-2-thienyl)méthylène]-2,4,6(1H,3H,5H)-pyrimidinetrione |
| (4) | 1,3-diméthyl-5-[(5-méthyl-2-furyl)méthylène]-2,4,6(1H,3H,5H)-pyrimidinetrione |
| (5) | 1-(4-méthoxyphényl)-5-[(5-méthyl-2-furyl)méthylène] -2,4,6(1H,3H,5H)-pyrimidinetrione |
| (6) | 1-(4-chlorophényl)-5-[(5-méthyl-2-furyl)méthylène] -2,4,6(1H,3H,5H)-pyrimidinetrione |
| (7) | 1-(4-isopropylphényl)-5-[(5-méthyl-2-furyl)méthylène]-2,4,6(1H,3H,5H)-pyrimidinetrione |
| (8) | 1-allyl-5-[(5-méthyl-2-furyl)méthylène]-2-thioxodihydro-4,6(1H,5H)-pyrimidinetrione |
| (9) | 5-(2-furylméthylène)-1-phenyl-2,4,6(1H,3H,5H)-pyrimidinetrione |
| (10) | 5-(2-furylméthylène)-1,3-diméthyl-2,4,6(1H,3H,5H)-pyrimidinetrione |
| (11) | 5-[(5-méthyl-2-furyl)méthylène]-1-(4-méthylphényl) -2,4,6(1H,3H,5H)-pyrimidinetrione |
| (12) | 1-(3-méthoxyphényl)-5-[(5-méthyl-2-furyl)méthylène] -2,4,6(1H,3H,5H)-pyrimidinetrione |
| (13) | 1-(4-éthoxyphényl)-5-[(5-méthyl-2-furyl)méthylène] -2,4,6(1H,3H,5H)-pyrimidinetrione |
| (14) | 1-(4-bromophényl)-5-[(5-méthyl-2-thiényl)méthylène] -2,4,6(1H,3H,5H)-pyrimidinetrione |
| (15) | 1-(4-chlorophényl)-5-[(5-méthyl-2-thiényl)méthylène] -2,4,6(1H,3H,5H)-pyrimidinetrione |
| (16) | 1-(4-fluorophényl)-5-[(5-méthyl-2-thiényl)méthylène] -2,4,6(1H,3H,5H)-pyrimidinetrione |
| (17) | 1-(3,4-diméthylphényl)-5-[(5-méthyl-2-furyl)méthylène]-2,4,6(1H,3H,5H)-pyrimidinetrione |
| (18) | 1-(2-furylméthyl)-5-[(5-méthyl-2-furyl)méthylène] -2,4,6(1H,3H,5H)-pyrimidinetrione |
| (19) | 1-(4-éthylphényl)-5-[(5-méthyl-2-furyl)méthylène] -2,4,6(1H,3H,5H)-pyrimidinetrione |
| (20) | 1-butyl-5-[(5-méthyl-2-thienyl)méthylène] -2,4,6(1H,3H,5H)-pyrimidinetrione |

Les composés préférés qui sont cités dans le tableau ci-dessus sont connus en tant que tels et sont décrits en particulier dans les publications suivantes : Vvedenskii, VM., Khimiya Geterotsiklicheskikh Soedinenii, 1969, 6, 1092-1095 ; El-Badawi, M., Journal of Science, 1990, 14(2), 581-600. Cependant, leur activité insecticide n'a jamais été mise en évidence dans l'art antérieur.

Du fait de l'activité inhibitrice de l'AChE que présentent les composés dérivés de pyrimidinetrione de formule générale (I) selon la présente Invention, ces composés peuvent également être utilisés pour la préparation d'un médicament destiné à traiter les troubles associés à un dysfonctionnement de l'AChE et en particulier les maladies neurodégénératives telles que la maladie d'Alzheimer.En effet, les composés de l'Invention ont montré une activité inhibitrice sur l'AChE humaine recombinante.

Selon un deuxième aspect, l'Invention a pour objet l'utilisation d'au moins un composé de formule générale (I) en tant qu'insecticide.

Tous les composés de formule générale (I) préférés tels que définis précédemment peuvent être utilisés en tant qu'insecticides.

Les composés de formule générale (I) peuvent être utilisés pour contrôler le développement des populations de différents types d'insectes, notamment du genre *Aedes, Culiseta, Ochlerotatus, Uranoteania, Anopheles* ou *Culex,* et de préférence du genre *Anopheles* ou *Culex,* notamment pour la désinfestation de zones habitées ou plus généralement de zones où s'exerce une activité humaine telles que des zones agricoles par exemple.

Parmi les insectes du genre *Anopheles* ou *Culex,* on peut en particulier citer les espèces choisies parmi *Culex deserticola, Culex hortensis, Culex pipiens pipiens, Culex pipiens, Culex pipiens quinquefasciatus, Culex pipiens molestus, Culex tigripes, Anopheles albimanus, Anopheles arabiensis, Anopheles gambiae, Anopheles hyrcanus, Anopheles subpicus* et, *Anopheles sundaicus.*

Selon une forme préférée de l'Invention, les composés de formule générale (I) sont utilisés sur des populations d'insectes qui ont développé une résistance aux insecticides organophosphorés ou aux carbamates. En effet, les Inventeurs ont constaté de façon surprenante que si l'activité de ces composés sur des populations d'insectes sensibles aux carbamates et aux organophosphorés est comparable ou même inférieure à celle de ces insecticides connus de longue date, ces composés sont largement plus actifs sur les populations d'insectes résistants à ces insecticides conventionnels.

Selon une autre forme préférée, les composés de formule générale (I) selon la présente Invention sont utilisés en association avec au moins un autre insecticide classique permettant ainsi de traiter des zones dans lesquelles co-existent des populations d'insectes plus ou moins sensibles ou résistants aux différents insecticides conventionnels. L'association d'au moins un composé de formule générale (I) et d'au moins un insecticide choisi parmi les carbamates et les organophosphorés constitue un autre objet de l'Invention.

L'application peut être effectuée d'une manière classique, par exemple par arrosage, pulvérisation, aspersion, diffusion, badigeonnage, désinfection à sec, désinfection par voie humide, désinfection en immersion, désinfection en suspension ou incrustation.

Du fait des propriétés insecticides que présentent les dérivés de pyrimidinetrione de formule générale (I) selon la présente Invention, ces composés peuvent également être utilisés en tant que produit comparatif dans la mise en oeuvre de tests d'évaluation de propriétés insecticides.

Pour des questions pratiques d'utilisation, les composés de formule générale (I) selon la présente Invention peuvent se présenter sous forme de compositions.

C'est ainsi que selon un troisième aspect, l'Invention a pour objet une composition insecticide comprenant au moins un composé de formule générale (I) en association avec un support approprié à l'utilisation de la composition en tant qu'insecticide.

Dans la composition selon la présente Invention, les composés actifs sont mélangés avec un support solide ou sont dissous ou dispersés dans un support liquide. Les composés peuvent également être associés à des substances auxiliaires telles que des émulsifiants, des agents de mouillage, des agents de dispersion et des stabilisants.

Les composés dérivés de pyrimidinetrione de formule générale (I) et les compositions les contenant selon la présente Invention peuvent être incorporés aux formulations classiques telles que solutions, émulsions, suspensions, poudres, mousses, pâtes, granulés, aérosols, etc. Lesdites formulations étant préparés par des techniques classiques bien connues de l'homme du métier.

Selon une forme préférée, la composition insecticide de l'Invention comprend en outre au moins un autre composé doué d'activité insecticide, et en particulier un composé insecticide choisi parmi les organophosphorés et les carbamates.

Par ailleurs, la composition insecticide peut en outre contenir une ou plusieurs autres substances actives telles que par exemple les acaricides, les fongicides, les bactéricides, les engrais, les fertilisants, etc.

Les compositions selon l'Invention sont efficaces contre les populations d'insectes de sensibilité normale ou résistantes aux organophosphorés ou aux carbamates, de même qu'à tous les stades de développement des insectes. En effet, l'activité acétylcholinestérase étant requise pour une transmission correcte de l'influx nerveux, les composés de l'Invention sont inhibiteurs de l'AChE (sauvage ou mutée G119S) et sont donc actifs dès que l'activité de l'enzyme est requise (les larves sensibles meurent dès l'éclosion donc au stade L1 de développement). '

Les formes d'application telles que décrites précédemment concernant l'utilisation des dérivés de pyrimidinetrione de formule générale (I) en tant qu'insecticides (arrosage, pulvérisation, etc) sont également applicables aux compositions les comprenant, que celles-ci contiennent ou non en outre d'autres substances actives ou auxiliaires.

Enfin selon un dernier aspect, l'Invention a pour objet un kit insecticide comprenant au moins une composition telle que définie précédemment associée à un moyen de diffusion permettant l'application de ladite composition sur un territoire constitué d'une ou plusieurs zones agricole et/ou d'habitation et/ou forestière et/ou marécageuse et/ou de steppe et/ou de savane.

La présente Invention sera mieux comprise à l'aide du complément de description qui va suivre, qui se réfère à des exemples de mise en évidence de la propriété d'inhibition de l'AChE de plusieurs composés dérivés de pyrimidinetrione de formule générale (I) conformes à l'Invention, de mise en évidence de la propriété insecticide de ces composés, ainsi qu'à la préparation d'une composition comprenant ces composés.

### EXEMPLE 1 : PROPRIETE D'INHIBITION DE L'ACTIVITE AChE DES COMPOSES DE FORMULE GENERALE (I)

Les composés dérivés de pyrimidinetrione de formule générale (I) selon l'Invention qui sont regroupés dans le tableau II ci-dessous ont été testés pour leur activité inhibitrice de l'AChE. Ces composés sont tous commercialisés par la société Chembridge Corporation (San Diego, USA).

**TABLEAU II**

| Composés | Noms |
|---|---|
| (1) | 5-[(5-méthyl-2-furyl)méthylène]-1-phényl-2,4,6(1H,3H,5H)-pyrimidinetrione |
| (2) | 1-(3-méthylphényl)-5-[(5-méthyl-2-thienyl)méthylène]-2,4,6(1H,3H,5H)-pyrimidinetrione |
| (3) | 1-(3-méthoxyphenyl)-5-[(5-méthyl-2-thienyl)méthylène]-2,4,6(1H,3H,5H)-pyrimidinetrione |
| (4) | 1,3-diméthyl-5-[(5-méthyl-2-furyl)méthylène]-2,4,6(1H,3H,5H)-pyrimidinetrione |
| (5) | 1-(4-méthoxyphényl)-5-[(5-méthyl-2-furyl)méthylène]-2,4,6(1H,3H,5H)-pyrimidinetrione |
| (6) | 1-(4-chlorophényl)-5-[(5-méthyl-2-furyl)méthylène] -2,4,6(1H,3H,5H)-pyrimidinetrione |
| (7) | 1-(4-isopropylphényl)-5-[(5-méthyl-2-furyl)méthylène] -2,4,6(1H,3H,5H)-pyrimidinetrione |
| (8) | 1-allyl-5-[(5-méthyl-2-furyl)méthylène]-2-thioxodihydro-4,6(1H,5H) -pyrimidinetrione |
| (9) | 5-(2-furylméthylène)-1-phenyl-2,4,6(1H,3H,5H)-pyrimidinetrione |
| (10) | 5-(2-furylméthylène)-1,3-diméthyl-2,4,6(1H,3H,5H)-pyrimidinetrione |
| (11) | 5-[(5-méthyl-2-furyl)méthylène]-1-(4-méthylphényl) -2,4,6(1H,3H,5H)-pyrimidinetrione |
| (12) | 1-(3-méthoxyphényl)-5-[(5-méthyl-2-furyl)méthylène] -2,4,6(1H,3H,5H)-pyrimidinetrione |
| (13) | 1-(4-éthoxyphényl)-5-[(5-méthyl-2-furyl)méthylène] -2,4,6(1H,3H,5H)-pyrimidinetrione |
| (14) | 1-(4-bromophényl)-5-[(5-méthyl-2-thiényl)méthylène] -2,4,6(1H,3H,5H)-pyrimidinetrione |
| (15) | 1-(4-chlorophényl)-5-[(5-méthyl-2-thiényl)méthylène] -2,4,6(1H,3H,5H)-pyrimidinetrione |
| (16) | 1-(4-fluorophényl)-5-[(5-méthyl-2-thiényl)méthylène] -2,4,6(1H,3H,5H)-pyrimidinetrione |
| (17) | 1-(3,4-diméthylphényl)-5-[(5-méthyl-2-furyl)méthylène] -2,4,6(1H,3H,5H)-pyrimidinetrione |
| (18) | 1-(2-furylméthyl)-5-[(5-méthyl-2-furyl)méthylène] -2,4,6(1H,3H,5H)-pyrimidinetrione |
| (19) | 1-(4-éthylphényl)-5-[(5-méthyl-2-furyl)méthylène] -2,4,6(1H,3H,5H)-pyrimidinetrione |
| (20) | 1-butyl-5-[(5-méthyl-2-thienyl)méthylène] -2,4,6(1H,3H,5H)-pyrimidinetrione |

Les propriétés inhibitrices des composés du tableau I qui précède sont testées sur l'activité AChE en utilisant soit une AChE recombinante normale ou mutée G119S, soit des extraits de larves de moustiques sensibles (porteurs de l'AChE normale) ou résistants (porteurs de l'AChE mutée G119S) aux organophosphorés et carbamates.

L'activité AChE est mesurée en utilisant la méthode décrite par Bourguet et al., Pest Biochem. Physiol., 1996, 55, 122-128 et qui fait référence à la méthode spectroscopique décrite par Ellman GL. et al. (Biochem Pharmacol, 1961, 7, 88-95) en utilisant l'acétylthiocholine comme substrat.

### 1) Propriété d'inhibition de l'activité d'une AchE recombinante

Les propriétés inhibitrices des composés selon l'Invention sur l'activité d'une AChE1 de *Culex pipiens* recombinante normale ou mutée G119S est testée selon le protocole décrit par Weill et al., Nature, 2003, 423, 136-137.

Chaque composé selon l'Invention est testé à différentes concentrations sur des lysats de cellules S2 de Drosophile transfectées avec un vecteur d'expression vide ou exprimant l'AChEl normale ou mutée. Après expression, les cellules sont centrifugées 15 minutes à 14000 rpm et sont ensuites lysées dans un tampon phosphate 0.25 M contenant 1 % de Triton X100.

100 µl de surnageant provenant de la centrifugation des lysats cellulaires 3 minutes à 10000 rpm est incubé pendant 15 minutes avec 10 µl de chaque composé selon l'Invention à diverses concentrations. L'activité d'inhibiteurs connus de l'AChE, à savoir de la tacrine et du propoxur (insecticide du type carbamate) est également testée en parallèle sur l'activité AChE (la tacrine et le propoxur sont disponibles dans le commerce chez ICN Biomedical et Bayer AG respectivement).

Pour chaque composé testé, on détermine la concentration conduisant à 50 % d'inhibition de l'activité AChE (CI50 en µM). L'efficacité des différents composés sur l'activité AChE mutée relativement à celle de l'AChE normale est estimée par le rapport sensible/insensible.

Les résultats obtenus (CI50 en µM et rapport sensible/insensible) sont résumés ci-dessous dans le tableau III.

**TABLEAU III**

| Composés | AChE1 mutée (protéine insensible) | AChE1 normale (protéine sensible) | Rapport sensible/insensible |
|---|---|---|---|
| Tacrine | 1,57 | 0,65 | 0,41 |
| (1) | 23 | 34 | 1,47 |
| (2) | 43 | 39 | 0,90 |
| (3) | 62 | 102 | 1,65 |
| (4) | 31 | 73 | 2,40 |
| (5) | 22 | 30 | 1,36 |
| (6) | 43 | 70 | 1,63 |
| (7) | 72 | 128 | 1,78 |
| (8) | 29 | 61 | 2,08 |
| (9) | 737 | 1394 | 1,89 |
| (10) | 357 | 1189 | 3,34 |
| (11) | 3,5 | 4,5 | 1,27 |
| (12) | 3,5 | 3,4 | 0,96 |
| (13) | 2,9 | 7,1 | 2,40 |
| (14) | 11,1 | 9,6 | 0,87 |
| (15) | 8,7 | 8,7 | 1,00 |
| (16) | 8,6 | 17,1 | 1,99 |
| (17) | 8,5 | 15,7 | 1,85 |
| (18) | 4,8 | 10,1 | 2,10 |
| (19) | 3,4 | 1,9 | 0,58 |
| (20) | 15,5 | 12,1 | 0,78 |
| Propoxur | 6348 | 9 | 0,0014 |

### 2) Propriété d'inhibition sur des extraits de larves de moustiques

Les larves de moustiques *Culex pipiens* sensibles ou résistantes au propoxur sont broyées dans 400 µl de tampon phosphate 0,25M contenant 1 % de Triton X100. Les homogénats sont centrifugés 3 minutes à 10000 rpm.

L'inhibition de l'activité AChE1 est mesurée sur le surnageant selon la méthode décrite au paragraphe 1), à savoir que 100 µl de surnageant est incubé pendant 15 minutes avec 10 µl de composés selon l'Invention à diverses concentrations. L'activité des inhibiteurs connus de l'AChE, à savoir de la tacrine et du propoxur est également testée en parallèle. L'efficacité des différents composés sur les larves résistantes relativement aux larves sensibles est estimée par le rapport sensibles/résistantes.

Les résultats obtenus (CI50 en µM et rapport sensibles/résistantes) sont résumés ci-dessous dans le tableau IV.

**TABLEAU IV**

| Composés | Larves résistantes | Larves sensibles | Rapport sensibles/résistantes |
|---|---|---|---|
| Tacrine | 5,5 | 3,5 | 0,6 |
| (1) | 6,2 | 29,4 | 4,7 |
| (2) | 28 | 442 | 15,5 |
| (3) | 33 | 118 | 3,5 |
| (4) | 7,5 | 74,3 | 9,8 |
| (5) | 81 | 592 | 7,3 |
| (6) | 27 | 330 | 12,0 |
| (7) | 21 | 132 | 6,1 |
| (8) | 51 | 91,6 | 1,8 |
| (11) | 14 | 111 | 7,7 |
| (12) | 6 | 54 | 8,5 |
| (13) | 89 | 236 | 2,7 |
| (14) | 142 | 2121 | 15,0 |
| (15) | 177 | 1220 | 6,9 |
| (16) | 22 | 490 | 22,5 |
| (17) | 35 | 424 | 12,2 |
| (18) | 37 | 260 | 7,1 |
| (19) | 19 | 312 | 16,7 |
| (20) | 152 | 153,0 | 1,0 |
| Propoxur | 31820 | 4,9 | 0,00015 |

L'ensemble de ces résultats montre que les composés de formule (I) selon l'Invention sont capables d'inhiber efficacement l'activité AChE, et en particulier l'activité d'une AChE mutée.

### EXEMPLE 2: PROPRIETE INSECTICIDE DES COMPOSES DE FORMULE GENERALE (I)

L'activité insecticide des composés selon l'Invention désignés dans le tableau IV ci-dessous est testée sur des larves de moustiques *Culex pipiens* sensibles ou résistants à l'action du propoxur.

Les larves résistantes ou sensibles au propoxur sont incubées en plaques de 24 puits, à raison de 15 larves de stade 4 par puits, dans 1 ml d'eau du robinet contenant différentes concentrations de composés allant de 0.31 à 50 µg/ml.

La mortalité larvaire est mesurée après 24 heures d'incubation. Pour chaque composé, la dose conduisant à 50 % de mortalité (DL50 en µM) est estimée à l'aide du programme log-Probit (Raymond, M. (1985). Présentation d'un programme Basic d'analyse log-probit pour micro-ordinateur. Cahiers ORSTOM, série Entomologie médicale et Parasitologie, 23(2), 117-121; Raymond, M., Prato, G., & Ratsira, D. (1993). PROBIT, CNRS-UMII, Licence L93019. Avenix, 34680 St. Georges d'Orques, France.), en testant l'homogénéité de la dose-réponse. L'efficacité des différents composés sur les larves résistantes relativement aux larves sensibles est estimée par le rapport sensibles/résistantes.

Les résultats obtenus (DL50 en µM et rapport sensibles/résistantes) sont résumés ci-dessous dans le tableau V suivant :

**TABLEAU V**

| Composés | Larves résistantes | Larves sensibles | Rapport sensibles/résistantes |
|---|---|---|---|
| (1) | 143 | 310 | 2,2 |
| (2) | 170 | 455 | 2,7 |
| (3) | 122 | 389 | 3,2 |
| (4) | 200 | 576 | 2,9 |
| (5) | 206 | 300 | 1,5 |
| (6) | 192 | 762 | 4,0 |
| (8) | 123 | 223 | 1,8 |
| Propoxur | 2700 | 1,7 | 0,00063 |

Ces résultats démontrent clairement que les composés de formule I selon l'Invention sont efficaces en tant qu'insecticides, et en particulier sur des populations d'insectes qui sont résistants aux insecticides classiques du type propoxur.

### EXEMPLE 3 : PREPARATION D'UNE COMPOSITION INSECTICIDE SELON L'INVENTION

On introduit 42 mg du composé (1) dans 100 ml d'eau du robinet, puis le mélange est agité.

## Revendications

1. Utilisation d'au moins un composé répondant à la formule générale (I) suivante : 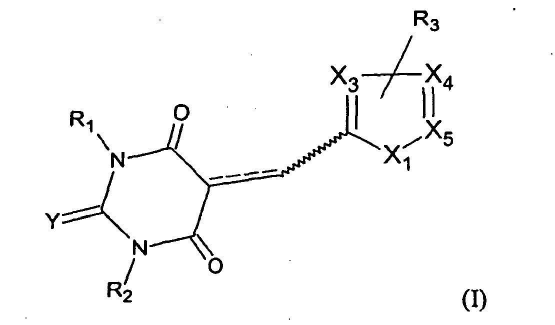 dans laquelle :
---- représente une liaison qui peut être une liaison simple ou une liaison double ;
R₁ représente un groupe choisi parmi un atome d'hydrogène et un groupe alkyle, saturé ou insaturé, linéaire, ramifié ou cyclique, en C₁-C₆, éventuellement substitué par un groupe hydroxyle ou un atome d'halogène ;
R₂ représente un groupe choisi parmi un groupe aryle éventuellement substitué et un groupe alkyle, saturé ou insaturé, linéaire, ramifié ou cyclique, en C₁-C₄, éventuellement substitué par un ou plusieurs groupements choisis parmi les atomes d'halogène, les groupements hydroxyles, les hétérocycles comprenant 1 à 5 atomes de carbone et 1 à 5 hétéro-atomes tels que l'oxygène, le soufre et l'azote ;
R₃ peut être en position 3, 4 ou 5 du cycle et représente un groupe choisi parmi un atome d'hydrogène et un groupe alkyle, saturé ou insaturé, linéaire, ramifié ou cyclique, en C₁-C₄, éventuellement substitué par un groupe hydroxyle ou un atome d'halogène ;
X₁ représente un atome choisi parmi le soufre, l'azote et l'oxygène ;
X₃, X₄ et X₅, identiques ou différents, sont choisis parmi un atome de carbone et un atome d'azote, deux atomes au plus parmi X₃, X₄ et X₅ étant des atomes d'azote ; et
Y représente un atome choisi parmi l'oxygène et le soufre,
en tant qu'inhibiteur de l'acétylcholinestérase.

2. Utilisation selon la revendication 1, **caractérisée en ce que** le composé répond à la formule générale (I) dans laquelle le cycle 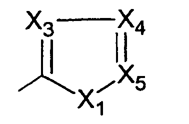 est choisi parmi 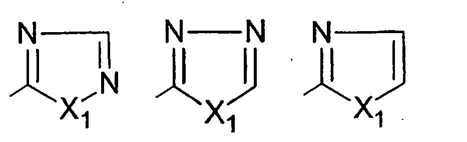 et 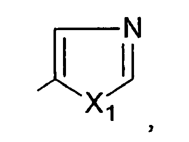 X₁ ayant la même signification que celle définie à la revendication 1.

3. Utilisation selon la revendication 1 ou 2, **caractérisée en ce que** le composé répond à la formule générale (I) dans laquelle R₁ représente un groupe alkyle en C₁-C₄.

4. Utilisation selon la revendication 3, **caractérisée en ce que** R₁ est choisi parmi un atome d'hydrogène et un groupe méthyle.

5. Utilisation selon la revendication 4, **caractérisée en ce que** R₁ représente un groupe méthyle.

6. Utilisation selon l'une quelconque des revendications précédentes, **caractérisée en ce que** le composé répond à la formule générale (I) dans laquelle R₂ représente un groupe aryle, éventuellement substitué par un groupement choisi parmi un alkyle en C₁-C₄ et un atome d'halogène.

7. Utilisation selon la revendication 6, **caractérisée en ce que** R₂ représente un phényle, éventuellement substitué par un groupement choisi parmi un alkyle en C₁-C₄ et un atome d'halogène.

8. Utilisation selon l'une quelconque des revendications 1 à 5, **caractérisée en ce que** le composé répond à la formule générale (I) dans laquelle R₂ est choisi parmi un méthyle, un allyle, un groupement furyl éventuellement substitué par un méthyle, un butyle, un phényle non substitué, un phényle substitué par un groupe méthyle en position méta, un phényle substitué par un groupe méthyle en position para, un phényle di-substitué en position méta et para par un méthyle, un phényle substitué par un éthyle en position para, un phényle substitué par un groupe méthoxy en position méta, un phényle substitué par un groupe méthoxy en position para, un phényle substitué par un éthoxy en position para, un phényle substitué par un isopropyle en position para, un phényle substitué par un atome de chlore en position para, un phényle substitué par un atome de brome en position para, un phényle substitué par un atome de fluor en position para.

9. Utilisation selon l'une quelconque des revendications précédentes, **caractérisée en ce que** le composé répond à la formule générale (I) dans laquelle X₁ est choisi parmi l'oxygène et le soufre.

10. Utilisation selon l'une quelconque des revendications précédentes, **caractérisée en ce que** le composé répond à la formule générale (I) dans laquelle X₃, X₄ et X₅ sont identiques et représentent tous un atome de carbone.

11. Utilisation selon l'une quelconque des revendications précédentes, **caractérisée en ce que** le composé répond à la formule générale (I) dans laquelle R₃ est choisi parmi un atome d'hydrogène et un groupe méthyle.

12. Utilisation selon l'une quelconque des revendications précédentes, **caractérisée en ce que** le composé répond à la formule générale (I) dans laquelle R₃ est en position 5 du cycle furanne, thiophène ou pyrrole.

13. Utilisation selon la revendication 1, **caractérisée en ce que** le composé répondant à la formule générale (I) est choisi parmi :
| Composés | Noms |
|---|---|
| (1) | 5-[(5-méthyl-2-furyl)méthylène]-1-phényl-2,4,6(1H,3H,5H)-pyrimidinetrione |
| (2) | 1-(3-méthylphényl)-5-[(5-méthyl-2-thienyl)méthylène]-2,4,6(1H,3H,5H)-pyrimidinetrione |
| (3) | 1-(3-méthoxyphenyl)-5-[(5-méthyl-2-thienyl)méthylène]-2,4,6(1H,3H,5H)-pyrimidinetrione |
| (4) | 1,3-diméthyl-5-[(5-méthyl-2-furyl)méthylène]-2,4,6(1H,3H,5H)-pyrimidinetrione |
| (5) | 1-(4-méthoxyphényl)-5-[(5-méthyl-2-furyl)méthylène] -2,4,6(1H,3H,5H)-pyrimidinetrione |
| (6) | 1-(4-chlorophényl)-5-[(5-méthyl-2-furyl)méthylène] -2,4,6(1H,3H,5H)-pyrimidinetrione |
| (7) | 1-(4-isopropylphényl)-5-[(5-méthyl-2-furyl)méthylène] -2,4,6(1H,3H,5H)-pyrimidinetrione |
| (8) | 1-allyl-5-[(5-méthyl-2-furyl)méthylène]-2-thioxodihydro-4,6(1H,5H) -pyrimidinetrione |
| (9) | 5-(2-furylméthylène)-1-phenyl-2,4,6(1H,3H,5H)-pyrimidinetrione |
| (10) | 5-(2-furylméthyléne)-1,3-diméthyl-2,4,6(1H,3H,5H)-pyrimidinetrione |
| (11) | 5-[(5-méthyl-2-furyl)méthylène]-1-(4-méthylphényl) -2,4,6(1H,3H,5H)-pyrimidinetrione |
| (12) | 1-(3-méthoxyphényl)-5-[(5-méthyl-2-furyl)méthylène] -2,4,6(1H,3H,5H)-pyrimidinetrione |
| (13) | 1-(4-éthoxyphényl)-5-[(5-méthyl-2-furyl)méthylène] -2,4,6(1H,3H,5H)-pyrimidinetrione |
| (14) | 1-(4-bromophényl)-5-[(5-méthyl-2-thiényl)méthylène] -2,4,6(1H,3H,5H)-pyrimidinetrione |
| (15) | 1-(4-chlorophényl)-5-[(5-méthyl-2-thiényl)méthylène] -2,4,6(1H,3H,5H)-pyrimidinetrione |
| (16) | 1-(4-fluorophényl)-5-[(5-méthyl-2-thiényl)méthylène] -2,4,6(1H,3H,5H)-pyrimidinetrione |
| (17) | 1-(3,4-diméthylphényl)-5-[(5-méthyl-2-furyl)méthyléne]-2,4,6(1H,3H,5H)-pyrimidinetrione |
| (18) | 1-(2-furylméthyl)-5-[(5-méthyl-2-furyl)méthylène]-2,4,6(1H,3H,5H)-pyrimidinetrione |
| (19) | 1-(4-éthylphényl)-5-[(5-méthyl-2-furyl)méthylène] -2,4,6(1H,3H,5H)-pyrimidinetrione |
| (20) | 1-butyl-5-[(5-méthyl-2-thienyl)méthylène] -2,4,6(1H,3H,5H)-pyrimidinetrione |

14. Utilisation d'au moins un composé de formule générale (I) telle que définie à l'une quelconque des revendications précédentes, en tant qu'insecticide.

15. Utilisation selon la revendication 14, **caractérisée en ce qu'**elle est destinée à contrôler le développement des populations d'insectes du genre *Anopheles* ou *Culex.*

16. Utilisation selon l'une quelconque des revendications 14 et 15, sur des populations d'insectes qui ont développé une résistance aux insecticides organophosphorés ou aux carbamates.

17. Composition insecticide comprenant au moins un composé de formule générale (I) telle que définie à l'une quelconque des revendications 1 à 13 en association avec un support approprié à l'utilisation de la composition en tant qu'insecticide.

18. Composition insecticide selon la revendication 17, **caractérisée en ce qu'**elle comprend en outre au moins un autre composé doué d'activité insecticide.

19. Composition insecticide selon la revendication 18, **caractérisée en ce que** l'autre composé doué d'activité insecticide est choisi parmi les organophosphorés et les carbamates.

20. Composition insecticide selon l'une quelconque des revendications 17 à 19, **caractérisée en ce qu'**elle contient en outre une ou plusieurs autres substances actives choisies parmi les acaricides, les fongicides, les bactéricides, les engrais et les fertilisants.

21. Kit insecticide comprenant au moins une composition telle que définie à l'une quelconque des revendications 17 à 20 associée à un moyen de diffusion permettant l'application de ladite composition sur un territoire constitué d'une ou plusieurs zones agricole et/ou d'habitation et/ou forestière et/ou marécageuse et/ou de steppe et/ou de savane.

22. Utilisation selon l'une quelconque des revendications 1 à 13, pour la préparation d'un médicament destiné à traiter les maladies neurodégénératives telles que la maladie d'Alzheimer.

## Claims

1. Use of at least one compound corresponding to the following general formula (I): 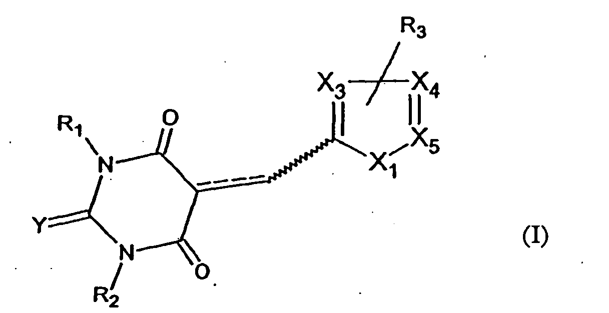 wherein:
---- denotes a bond which may be a single bond or a double bond;
R₁ denotes a group selected from among a hydrogen atom and a saturated or unsaturated, straight-chain, branched or cyclic C₁-C₆-alkyl group optionally substituted by a hydroxyl group or a halogen atom;
R₂ denotes a group selected from among an optionally substituted aryl group and a saturated or unsaturated, straight-chain, branched or cyclic C₁-C₄-alkyl group optionally substituted by one or more groups selected from among the halogen atoms, the hydroxyl groups, the heterocyclic groups comprising 1 to 5 carbon atoms and 1 to 5 heteroatoms such as oxygen, sulphur and nitrogen;
R₃ may be in the 3, 4 or 5 position of the ring and denotes a group selected from among a hydrogen atom and a saturated or unsaturated, straight-chain, branched or cyclic C₁-C₄-alkyl group optionally substituted by a hydroxyl group or a halogen atom;
X₁ denotes an atom selected from among sulphur, nitrogen and oxygen;
X₃, X₄ and X₅, which may be identical or different, are selected from among a carbon atom and a nitrogen atom, at most two of the atoms X₃, X₄ and X₅ being nitrogen atoms; and
Y denotes an atom selected from among oxygen and sulphur,
as an acetylcholinesterase inhibitor.

2. Use according to claim 1, **characterised in that** the compound corresponds to the general formula (I) wherein the cyclic group 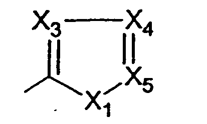 is selected from among 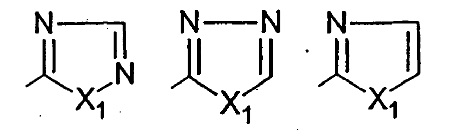 and 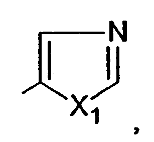 wherein
X₁ has the same meaning as defined in claim 1.

3. Use according to claim 1 or 2, **characterised in that** the compound corresponds to the general formula (I) wherein R₁ denotes a C₁-C₄-alkyl group.

4. Use according to claim 3, **characterised in that** R₁ is selected from among a hydrogen atom and a methyl group.

5. Use according to claim 4, **characterised in that** R₁ denotes a methyl group.

6. Use according to any one of the preceding claims, **characterised in that** the compound corresponds to the general formula (I) wherein R₂ denotes an aryl group optionally substituted by a group selected from among a C₁-C₄-alkyl and a halogen atom.

7. Use according to claim 6, **characterised in that** R₂ denotes a phenyl optionally substituted by a group selected from among a C₁-C₄-alkyl and a halogen atom.

8. Use according to any one of claims 1 to 5, **characterised in that** the compound corresponds to the general formula (I) wherein R₂ is selected from among a methyl, an allyl, a furyl group optionally substituted by a methyl, a butyl, an unsubstituted phenyl, a phenyl substituted by a methyl in the *meta* position, a phenyl substituted by a methyl group in the *para* position, a phenyl disubstituted in the *meta* and *para* position by a methyl, a phenyl substituted by an ethyl in the *para* position, a phenyl substituted by a methoxy group in the *meta* position, a phenyl substituted by a methoxy group in the *para* position, a phenyl substituted by an ethoxy in the *para* position, a phenyl substituted by an isopropyl in the *para* position, a phenyl substituted by a chlorine atom in the *para* position, a phenyl substituted by a bromine atom in the *para* position, a phenyl substituted by a fluorine atom in the *para* position.

9. Use according to any one of the preceding claims, **characterised in that** the compound corresponds to the general formula (I) wherein X₁ is selected from among oxygen and sulphur.

10. Use according to any one of the preceding claims, **characterised in that** the compound corresponds to the general formula (I) wherein X₃, X₄ and X₅ are identical and all represent a carbon atom.

11. Use according to any one of the preceding claims, **characterised in that** the compound corresponds to the general formula (I) wherein R₃ is selected from among a hydrogen atom and a methyl group.

12. Use according to any one of the preceding claims, **characterised in that** the compound corresponds to the general formula (I) wherein R₃ is in the 5 position of the furan, thiophene or pyrrole ring.

13. Use according to claim 1, **characterised in that** the compound corresponding to general formula (I) is selected from among:
| Compounds | Names |
|---|---|
| (1) | 5-[(5-methyl-2-furyl)methylene]-1-phenyl-2,4,6(1H,3H,5H)-pyrimidinetrione |
| (2) | 1-(3-methylphenyl)-5-[(5-methyl-2-thienyl)methylene]-2,4,6(1H,3H,5H)-pyrimidinetrione |
| (3) | 1-(3-methoxyphenyl)-5-[(5-methyl-2-thienyl)methylene]-2,4,6(1H,3H,5H)-pyrimidinetrione |
| (4) | 1,3-dimethyl-5-[(5-methyl-2-furyl)methylene]-2,4,6 (1H,3H,5H)-pyrimidinetrione |
| (5) | 1-(4-methoxyphenyl)-5-[(5-methyl-2-furyl)methylene]-2,4,6 (1H,3H,5H)-pyrimidinetrione |
| (6) | 1-(4-chlorophenyl)-5-[(5-methyl-2-furyl)methylene]-2,4,6 (1H,3H,5H)-pyrimidinetrione |
| (7) | 1-(4-isopropylphenyl)-5-[(5-methyl-2-furyl)methylene]-2,4,6 (1H,3H,5H)-pyrimidinetrione |
| (8) | 1-allyl-5-[(5-methyl-2-furyl)methylene]-2-thioxodihydro-4,6(1H,5H)-pyrimidinetrione |
| (9) | 5-(2-furylmethylene]-1-phenyl-2,4,6(1H,3H,5H)-pyrimidinetrione |
| (10) | 5-(2-furylmethylene]-1,3-dimethyl-2,4,6(1H,3H,5H)-pyrimidinetrione |
| (11) | 5-[(5-methyl-2-furyl)methylene]-1-(4-methylphenyl)-2,4,6(1H,3H,5H)-pyrimidinetrione |
| (12) | 1-(3-methoxyphenyl)-5-[(5-methyl-2-furyl)methylene]-2,4,6 (1H,3H,5H)-pyrimidinetrione |
| (13) | 1-(4-ethoxyphenyl)-5-[(5-methyl-2-furyl)methylene]-2,4,6(1H,3H,5H)-pyrimidinetrione |
| (14) | 1-(4-bromophenyl)-5-[(5-methyl-2-thienyl)methylene]-2,4,6(1H,3H,5H)-pyrimidinetrione |
| (15) | 1-(4-chlorophenyl)-5-[(5-methyl-2-thienyl)methylene]-2,4,6(1H,3H,5H)-pyrimidinetrione |
| (16) | 1-(4-fluorophenyl)-5-[(5-methyl-2-thienyl)methylene]-2,4,6(1H,3H,5H)-pyrimidinetrione |
| (17) | 1-(3,4-dimethylphenyl)-5-[(5-methyl-2-furyl)methylene]-2,4,6(1H,3H,5H)-pyrimidinetrione |
| (18) | 1-(2-furylmethyl)-5-[(5-methyl-2-furyl)methylene]-2,4,6 (1H,3H,5H)-pyrimidinetrione |
| (19) | 1-(4-ethylphenyl)-5-[(5-methyl-2-furyl)methylene]-2,4,6(1H,3H,5H)-pyrimidineuione |
| (20) | 1-butyl-5-[(5-methyl-2-thienyl)methylene]- 2,4,6(1H,3H,5H)-pyrimidinetrione |

14. Use of at least one compound of general formula (I) as defined in any one of the preceding claims, as an insecticide.

15. Use according to claim 14, **characterised in that** it is intended for controlling the development of populations of insects of the genus *Anopheles* or *Culex*.

16. Use according to any one of claims 14 and 15, on populations of insects that have developed a resistance to organophosphorus insecticides or carbamates.

17. Insecticidal composition comprising at least one compound of general formula (I) as defined in any one of claims 1 to 13 combined with a carrier which is suitable for the use of the composition as an insecticide.

18. Insecticidal composition according to claim 17, **characterised in that** it further comprises at least one other compound having an insecticidal activity.

19. Insecticidal composition according to claim 18, **characterised in that** the other compound having an insecticidal activity is selected from among the organophosphorus compounds and carbamates.

20. Insecticidal composition according to any one of claims 17 to 19, **characterised in that** it also contains one or more other active substances selected from among the acaricides, fungicides, bactericides, manures and fertilisers.

21. Insecticidal kit comprising at least one composition as defined in any one of claims 17 to 20 combined with distribution means enabling the composition to be applied to a territory consisting of one or more areas of agriculture and/or habitation and/or forestry and/or marsh and/or steppe and/or savannah.

22. Use according to any one of claims 1 to 13, for preparing a medicament intended for the treatment of neurodegenerative diseases such as Alzheimer's disease.

## Patentansprüche

1. Verwendung wenigstens einer Verbindung, wiedergegeben durch die folgende allgemeine Formel (I): 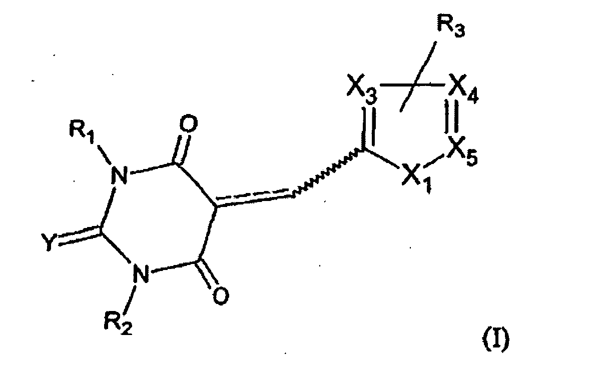 in der ---- eine Bindung darstellt, die eine Einfach- oder Doppelbindung sein kann;
R₁ eine Gruppe darstellt, ausgewählt aus einem Wasserstoffatom, und einer Alkylgruppe, gesättigt oder ungesättigt, geradkettig, verzweigt oder cyclisch, mit C₁-C₆, optional mit einer Hydroxylgruppe oder einem Halogenatom substituiert;
R₂ eine Gruppe darstellt, ausgewählt aus einer Arylgruppe, optional substituiert, und einer Alkylgruppe, gesättigt oder ungesättigt, geradkettig, verzweigt oder cyclisch, mit C₁-C₄, optional substituiert mit einer oder mehreren Gruppierungen,
ausgewählt aus Halogenatomen, Hydroxylgruppierungen, Heterocyclen, umfassend 1 bis 5 Kohlenstoffatome und 1 bis 5 Heteroatome, wie Sauerstoff, Schwefel und Stickstoff;
R₃ sich in Position 3, 4 oder 5 des Rings befinden kann und eine Gruppe darstellt, ausgewählt aus einem Wasserstoffatom und einer Alkylgruppe, gesättigt oder ungesättigt, geradkettig, verzweigt oder cyclisch, mit C₁-C₄, optional mit einer Hydroxylgruppe oder einem Halogenatom substituiert;
X₁ ein aus Schwefel, Stickstoff und Sauerstoff ausgewähltes Atom darstellt;
X₃, X₄, X₅ gleichartig oder verschieden, ausgewählt sind aus einem Kohlenstoffatom und einem Stickstoffatom, wobei höchstens zwei Atome unter X₃, X₄, X₅ Stickstoffatome sind; und
Y ein aus Sauerstoff und Schwefel ausgewähltes Atom darstellt,
als Acetylcholinesterasehemmer.

2. Verwendung nach Anspruch 1, **dadurch gekennzeichnet, dass** die Verbindung durch die allgemeine Formel (1) wiedergegeben wird, in der der Ring 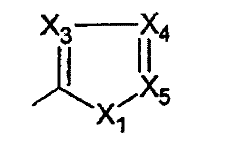 ausgewählt ist aus 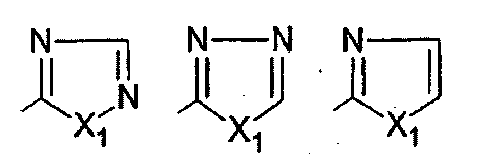 und 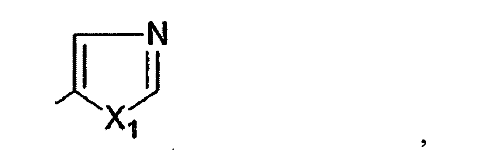 und X₁ dieselbe Bedeutung hat wie nach Anspruch 1 definiert.

3. Verwendung nach Anspruch 1 oder 2, **dadurch gekennzeichnet, dass** die Verbindung durch die allgemeine Formel (1) wiedergeben wird, in der R₁ eine C₁-C₄-Alkylgruppe darstellt.

4. Verwendung nach Anspruch 3, **dadurch gekennzeichnet, dass** R₁ ausgewählt ist aus einem Wasserstoffatom und einer Methylgruppe.

5. Verwendung nach Anspruch 4, **dadurch gekennzeichnet, dass** R₁ eine Methylgruppe darstellt.

6. Verwendung nach einem der vorhergehenden Ansprüche, **dadurch gekennzeichnet, dass** die Verbindung durch die allgemeine Formel (1) wiedergegeben wird, in der R₂ eine Arylgruppe darstellt, optional substituiert mit einer Gruppierung, ausgewählt aus einem C₁-C₄-Alkyl und einem Halogenatom.

7. Verwendung nach Anspruch 6, **dadurch gekennzeichnet, dass** R₂ ein Phenyl darstellt, optional substituiert mit einer Gruppierung, ausgewählt aus einem C₁-C₄-Alkyl und einem Halogenatom.

8. Verwendung nach einem der Ansprüche 1 bis 5, **dadurch gekennzeichnet, dass** die Verbindung durch die allgemeine Formel (1) wiedergegeben wird, in der R₂ ausgewählt ist aus Methyl, Allyl, einer Furylgruppierung, optional substituiert mit Methyl, Butyl, nicht substituiertem Phenyl, mit einer Methylgruppe in Metaposition substituiertem Phenyl, mit einer Methylgruppe in Paraposition substituiertem Phenyl, mit Methyl in Meta- und Paraposition di-substituiertem Phenyl, mit Ethyl in Metaposition substituiertem Phenyl, mit einer Methoxygruppe in Metaposition substituiertem Phenyl, mit einer Methoxygruppe in Paraposition substituiertem Phenyl, mit Ethoxy in Paraposition substituiertem Phenyl, mit Isopropyl in Paraposition substituiertem Phenyl, mit einem Chloratom in Paraposition substituiertem Phenyl, mit einem Bromatom in Paraposition substituiertem Phenyl, mit einem Fluoratom in Paraposition substituiertem Phenyl.

9. Verwendung nach einem der vorhergehenden Ansprüche, **dadurch gekennzeichnet, dass** die Verbindung durch die allgemeine Formel (1) wiedergegeben wird, in der X₁ ausgewählt ist aus Sauerstoff und Schwefel.

10. Verwendung nach einem der vorhergehenden Ansprüche, **dadurch gekennzeichnet, dass** die Verbindung durch die allgemeinen Formel (1) wiedergegeben wird, in der X₃, X₄ und X₅ identisch sind und alle ein Kohlenstoffatom darstellen.

11. Verwendung nach einem der vorhergehenden Ansprüche, **dadurch gekennzeichnet dass**, die Verbindung durch die die allgemeine Formel (1) wiedergegeben wird, in der R₃ ausgewählt ist aus einem Wasserstoffatom und einer Methylgruppe.

12. Verwendung nach einem der vorhergehenden Ansprüche, **dadurch gekennzeichnet dass**, die Verbindung durch die allgemeine Formel (1) wiedergegeben wird, in der sich R₃ in Position 5 des Furan-, Thiophen oder Pyrolrings befindet.

13. Verwendung nach Anspruch 1, **dadurch gekennzeichnet, dass** die durch die allgemeine Formel 1 wiedergegebene Verbindung, ausgewählt ist aus:
| Verbindung | Namen |
|---|---|
| 1 | 5-[(5-Methyl-2-furyl)methylen]-1-phenyl-2,4,6(1H,3H,5H)-pyrimidintrion |
| 2 | 1-(3-Methylphenyl)-5-[(5-methyl-2-thienyl)methylen]-2,4,6(1H,3H,5H)-pyrimidintrion |
| 3 | 1-(3-Methoxyphenyl)-5-[(5-methyl-2-thienyl)methylen]-2,4,6(1H,3H,5H)-pyrimidintrion |
| 4 | 1,3-Dimethyl-5-[(5-methyl-2-furyl)methylen] -2,4,6(1H,3H,5H)-pyrimidintrion |
| 5 | 1-(4-Methoxyphenyl)-5-[(5-methyl-2furyl)methylen] -2,4,6(1H,3H,5H)-pyrimidintrion |
| 6 | 1-(4-Chlorphenyl)-5-[(5-methyl-2-furyl)methylen] -2,4,6(1H,3H,5H)-pyrimidintrion |
| 7 | 1-(4-Isopropylphenyl)-5-[(5-methyl-2-furyl)methylen] -2,4,6(1H,3H,5H)-pyrimidintrion |
| 8 | 1-Allyl-5-[(5-methyl-2-furyl)methylen]-2-thioxodihydro-4,6(1H,5H)-pyrimidintrion |
| 9 | 5-(2-Furylmethylen)-1-phenyl-2,4,6(1H,3H,5H)-pyrimdintrion |
| 10 | 5-(2-Furylmethylen)-1,3-dimethyl-2,4,6(1H,3H,5H)-pyrimidintrion |
| 11 | 5-[(5-methyl-2-furyl)methylen]-1-(4-methylphenyl) -2,4,6(1H,3H,5H)-pyrimidintrion |
| 12 | 1-(3-Methoxyphenyl)-5-[(5-methyl-2-furyl)methylen] -2,4,6(1H,3H,5H)-pyrimidintrion |
| 13 | 1-(4-Ethoxyphenyl)-5-[(5-methyl-2-furyl)methylen] -2,4,6(1H,3H,5H)-pyrimidintrion |
| 14 | 1-(4-Bromphenyl)-5-[(5-methyl-2-thienyl)methylen] -2,4,6(1H,3H,5H)-pyrimidintrion |
| 15 | 1-(4-Chlorphenyl)-5-[(5-methyl-2-thienyl)methylen] -2,4,6(1H,3H,5H)-pyrimidintrion |
| 16 | 1-(4-Fluorphenyl)-5-[(5-methyl-2-thienyl)methylen] -2,4,6(1H,3H,5H)-pyrimidintrion |
| 17 | 1-(3,4-dimethylphenyl)-5-[(5-methyl-2-furyl)methylen] -2,4,6(1H,3H,5H)-pyrimidintrion |
| 18 | 1-(2-Furylmethyl)-5-[(5-methyl-2-furyl)methylen] -2,4,6(1H,3H,5H)-pyrimidintrion |
| 19 | 1-(4-Ethylphenyl)-5-[(5-methyl-2-furyl)methylen] -2,4,6(1H,3H,5H)-pyrimidintrion |
| 20 | 1-Butyl-5-[(5-methyl-2-thienyl)methylen] -2,4,6(1H,3H,5H)-pyrimidintrion |

14. Verwendung wenigstens einer, nach einem der vorhergehenden Ansprüche definierten Verbindung der allgemeinen Formel (1) als Insektizid.

15. Verwendung nach Anspruch 14, **dadurch gekennzeichnet dass**, sie zum Steuern der Entwicklung von Insektenpopulationen der Gattung *Anopheles* oder *Culex* bestimmt ist.

16. Verwendung nach einem der Ansprüche 14 und 15, für Insektenpopulationen, die eine Resistenz gegen organophosphorhaltige oder carbamathaltige Insektizide entwickelt haben.

17. Insektizidzusammensetzung, umfassend wenigstens eine Verbindung der allgemeinen Formel (1) nach einem der anspruche 1 bis 13 zusammen mit einem für die Verwendung der Zusammensetzung als Insektizid geeigneten Träger.

18. Insektizidzusammensetzung nach Anspruch 17, **dadurch gekennzeichnet, dass** sie zusätzlich wenigstens eine andere, mit einer insektiziden Eigenschaft ausgestattete Verbindung umfasst.

19. Insektizidzusammensetzung nach Anspruch 18, **dadurch gekennzeichnet, dass** die andere mit der insektiziden Eigenschaft ausgestattete Verbindung, ausgewählt ist aus Organophosphaten und Carbamaten.

20. Insektizidzusammensetzung nach einem der Ansprüche 17 bis 19, **dadurch gekennzeichnet, dass** sie zusätzlich eine oder mehrere andere aktive Substanzen enthält, auswählt aus Acariziden, Fungiziden, Bakteriziden, Düngern und Düngemitteln.

21. Insektizid-Kit, umfassend wenigstens eine nach den Ansprüchen 17 bis 20 definierte Zusammensetzung, die mit einem Diffusionsmittel verbunden ist, das die Applikation der Zusammensetzung auf ein Gebiet ermöglicht, das aus einem oder mehreren landwirtschaftlichen und/oder Wohn- und/oder Forst- und/oder Moor- und/oder Steppen- und/oder Savannen-Gebieten gebildet wird.

22. Verwendung nach einem der Ansprüche 1 bis 13, zum Herstellen eines Arzneimittels zum Behandeln neurodegenerativer Erkrankungen wie Alzheimer.
